# EUROPEAN PATENT APPLICATION

(11) **EP 1 918 025 A1**
(43) Date of publication of application: **07.05.2008**
(21) Application number: 07254324.2
(22) Date of filing: 31.10.2007
(51) Int. Cl.: B01L 3/18, G01N 1/04, G01N 27/447

(54) **Non-metallic laboratory implement and method of its use**

(30) Priority: 02.11.2006 US 592550
(71) Applicant: Bel-Art Products, Inc., Pequannock, New Jersey 07440 (US)
(72) Inventor: Yaniv, Abraham, Tel Aviv 69495 (IL); Gazit, Arnona, Tel Aviv 69362 (IL); Thom, Paul, Butler, New Jersey 07405 (US); Landsberger, David, Caldwell, New Jersey 07006 (US); Gomes, Francis, Lincoln Park, New Jersey 07036 (US)
(74) Representative: Green, Mark Charles

(57) **Abstract**

A laboratory implement (10) consisting of a body formed with at least a blade (26) at one end thereof is used for handling biological samples. The blade is provided with photoluminescent qualities, so as to emit visible light when subjected to ultraviolet light. In the method, the implement is positioned in a hand of the user with the blade facing the biological samples. The samples are cut by the blade in darkness under ultraviolet light, in such a manner that the blade is visible to an operator.

## Description

### FIELD OF THE INVENTION

This invention relates in general to laboratory implements, more specifically the invention relates to non-metallic laboratory implements adapted for handling biological samples and a method of its use.

### BACKGROUND OF THE INVENTION

Electrophoresis is the most common method in biologic technology for separating and analyzing nucleotides-based structures such as DNA, RNA, and protein. Macromolecules of DNA, RNA and protein are separated according to the size of each molecule by means of electric current which pulls the differently sized molecules across a colloidal gel structure at velocities inversely proportional to the mass of each molecule.

The liquid solution is poured into a tray containing a comb. The gel cools and solidifies and the comb is removed, leaving a series of spaced apart slots. A nucleotide-based solution, that is, a DNA, RNA, or protein solution, is then mixed with stain and inserted into at least one slot.

For DNA and RNA molecules, mainly a solution with 1 % agarose is used, though in some instances up to 2% agarose is used or the agarose is substituted with acrylamide solution. The solution is in a liquid state when heated but cools to form a colloidal gel structure such that particles are suspended in a colloidal dispersion. It is weak, easily breakable, and may be difficult to work with efficiently.

Included in the agarose solution is a low concentration of ethidium bromide, or another dye for staining the DNA. The dye fluoresces due to contents of some sort of fluorescent material and is visible under ultraviolet light. Without the stain, the DNA molecules would be virtually invisible in the gel. The stain allows for viewing until ultraviolet light of amounts of about 1 nanogram. Depending on the dye, the wavelengths of UV light used range from 254 to 366 nanometers.

The electrophoresis apparatus is then connected to a power source and the nucleotide fragments, which have a net negative charge, move across the gel away from the negatively charged anode and towards the positively charged cathode. The electrophoresis process is typically stopped when the smallest (i.e. fastest moving) molecule has moved almost completely across the gel. The distance moved for each molecule is a function of the size of the molecule. As there may be millions of pieces, some molecules may be of the same or similar size, although not identical in structure. Therefore, the degree of movement is indistinguishable and will require further and more precise separation.

In order to facilitate viewing the UV-stained molecules, the gel is placed onto a UV transilluminator as commonly known in the art. A transilluminator generally includes a housing having a horizontal top wall. The top wall has therein a UV-transmissible screen or window preferably made of an expensive purple filter glass which transmits UV light.

Pluralities of UV light sources are supported within the housing for transmitting UV light through the window. The transilluminator produces a substantially uniform level of UV light intensity across the UV-transmissible window. The screen or window of the transilluminator typically comprises a purple filter glass which blocks all light except that within a narrow range centered around the specific UV region which causes the fluorescence of ethidium bromide or other stain bound to DNA or RNA. The screen or filter glass is an expensive component of the transilluminator. Physical damage to the glass typically occurs when researchers use sharp, metallic conventional laboratory implements to cut out and manipulate DNA-containing bands of gel with the gel being illuminated on the glass. These metallic implements, such as sharp, metallic scalpels, are typically used by an operator during this procedure. Such damage to the glass surface can affect background UV transmission through the glass. The scratches to the glass surface left by the metallic scalpels result in clouding of the glass or in high levels of background fluorescence. All of the above substantially impair the accuracy of the procedures related to the DNA-containing bands of gel.

A typical cutting implement for use in this procedure is a common metallic laboratory scalpel known in the art. Such metallic scalpels are practically invisible in darkness under UV light so as to hinder the process of handling DNA and other biological samples. Use of the sharp metallic scalpels damage expensive screens of transilluminators. The metallic scalpel may contain molecules of DNA, RNA, or proteins, and thus, contaminate the samples. Furthermore utilization of a metal scalpel having a vertically oriented blade makes it difficult for a user to make the proper incisions without scratching, damaging, and ultimately destroying the expensive screens of UV transillminators. The high cutting angle of the typical scalpel and difficult viewing of its blade in UV light also make vision of the gel to be cut difficult, thereby decreasing the accuracy of the procedure. The close proximity of space between the screen and the gel makes it difficult to produce accurate incisions and the knife is always no more than a short distance from scratching the surface of the transilluminator or protective screen.

Thus, it has been a long-felt and unsolved need to provide a laboratory implement adapted for handling biological samples which is easily viewable under UV light and does not scratch or damage the sensitive and expensive filters or screens of the UV transilluminators. It has been a further need to provide a single use laboratory implement for handling biological samples which prevents cross-contamination and does not contain DNA, RNA, or protein molecules. The implement which is easily manipulated in narrow areas and does not obstruct the users view of the materials being cut or manipulated.

### SUMMARY OF THE INVENTION

One aspect of the invention provides a non-metallic laboratory implement which consists of a body having at least a gripping portion and a blade spaced from each other, with the blade being positioned at an angle to a longitudinal axis to the gripping portion. At least one photoluminescent substance is being dispersed within a material medium used in manufacturing said blade, so as to make it visible under ultraviolet light.

As to another aspect of the invention, the body further comprises a transition portion with the transition portion being formed between the blade and the gripping portion and a spatula being provided at an end of the body opposite to the blade. At least one photoluminescent substance is dispersed within the material medium of the spatula, so as to make it visible under ultraviolet light. The photoluminescent substance can be uniformly dispensed within the dispersing medium of the material over the entire body. In this manner, the entire body of the implement is visible under ultraviolet light. As to further aspects of the invention, the implement is manufactured in a nuclease-free manner, so that traces of nuclease (DNase and RNase) will not contaminate the isolated DNA and RNA with nucleases. As to still another aspect of the invention, a method of handling biological samples by means of an implement consisting of a body formed with at least a blade at one end thereof is being provided. The blade is formed having photoluminescent qualities and is capable of emitting visible light when subject to ultraviolet light. Said method is conducted in darkness under ultraviolet light and comprises the following steps.

The implement is initially positioned in a hand of a user, so as to face a sample containing biological samples. The blade emitting the visible light is observed by an operator. Then, the biological samples are being cut by the blade and the cut biological samples are being manipulated.

### Brief Description of the Drawings

FIGURE 1 is a front elevational view of a laboratory implement;

FIGURE 2 is a top plan view thereof;

FIGURE 3 is a bottom plan view thereof;

FIGURE 4 is a left hand side elevational view thereof;

FIGURE 5 is a right hand side elevational view thereof;

FIGURE 6 is a rear elevational view thereof;

FIGURE 7 is a sectional view according to section plane 7-7 of Figure 6;

FIGURE 8 a view illustrating one application of the laboratory implement of the invention;

FIGURE 9 is a view illustrating another use of the implement; and

FIGURE 10 is a view illustrating a further use of the implement.

### DESCRIPTION OF THE EMBODIMENTS OF THE INVENTION

These features are by design so as to allow the tool to be comfortably used in the narrow space between the agar gel and a protective screen.

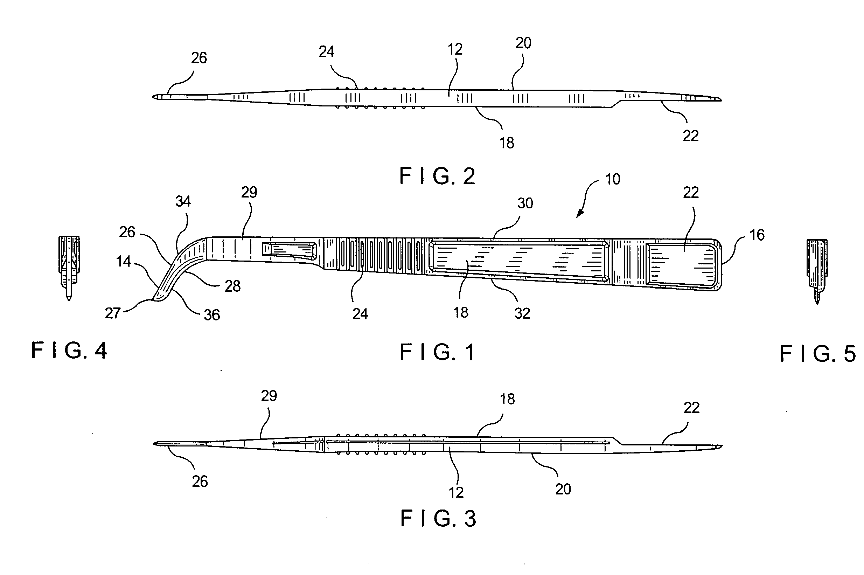

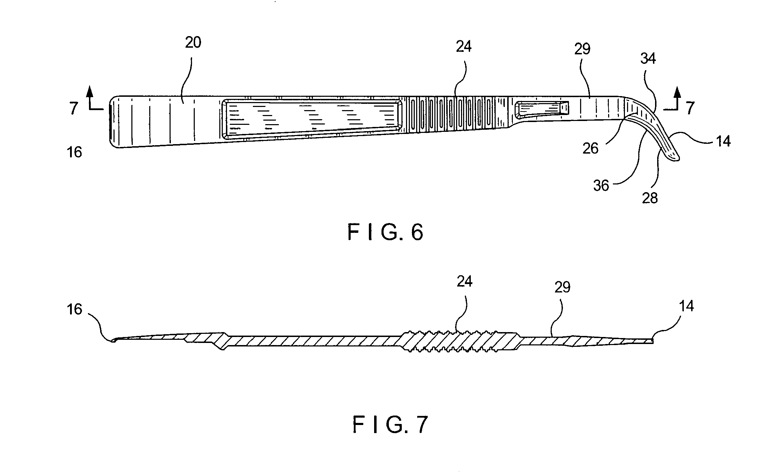

Referring now to FIGS 1-7, illustrating a non-metallic disposable laboratory implement 10 of the invention which consists of a body 12 having a front face 18, a rear face 20 disposed between a proximal end 14 and a distal end 16. A top edge 30 and a bottom edge 32 extend longitudinally along the body. A blade 26 is provided at the proximal end 14, whereas a recess or spatula 22 is formed within the front face 18, so as to extend from the distal end 16 toward a gripping area 24. The blade 26 is separated from the gripping area 24 by the transitional part 29. The blade 26 is positioned at an angle to the longitudinal axis of the body and is formed by a top curvature 34 and a bottom curvature 36 joined together at the tip 27. As clearly illustrated in FIGs 1 and 6, the top curvature 34 is connected to the top edge 30. On the other hand, the bottom curvature 36 is an extension of the bottom edge 32. The cutting region 28 of the blade forms a part of the bottom curvature 36 and extends from the transitional part 29 to the tip area 27. A gripping area 24 extends rearward from the transitional part 29 and is provided with a plurality of serrations developed on the front face 18 and the rear face 20 of the body of the implement. In this manner easy gripping of the implement is assured for a user with reasonably dexterity. The width of the body increases toward the distal end, so as to accommodate the recessed, flattened spatula 22 positioned in this area.

The implement is preferably made of a polymer plastic material such as polystyrene or the like to have sufficient stiffness to cut or otherwise manipulate biological samples, but resilient/soft enough to prevent scratches to the screen of a transilluminator. The implement is preferably integrally molded into a unitary structure during the manufacturing process. However, alternatively parts of the implement such as the blade 26, spatula 22, etc., may be formed separately and joined together through ultrasonic welding, adhesive, or other known joining techniques.

It is well known that some biological samples, such as nucleotide structures, for example, are eluted across the gel and practically invisible without UV light being utilized. Thus, standard procedures for removing and manipulating these biological samples are difficult and leave room for error. Another serious factor which typically negates the ability to conduct accurate work is the difficulty for the operator to see the prior art metallic tools or scalpels during such procedures conducted in darkness, even when working under UV light.

An essential feature of the invention is that the implement 10 can be totally or partially viewed by an operator in darkness under UV light. To achieve this function the body 12 of the implement is formed containing photoluminescent substances adapted to absorb light in the ultraviolet and violet region of the electromagnetic spectrum and to re-emit light, typically, in the blue region visible to an operator. In one embodiment of the invention the body 12 of the implement 10 is made from a plastic or other suitable dispersing medium having at least one substance with photoluminescent qualities being uniformly dispersed therein. In this manner, the photoluminescent substance is provided in proper quantities and thoroughly mixed with the medium of the plastic material to provide homogeneity of the photoluminescence function throughout the body of the implement. For example, such substance with photoluminescent qualities can be in the form of optical brighteners which are known for good solubility and thermal stability. The optical brightener compound in liquid form is added to the liquid plastic during manufacturing of the implement by injection or molding. Typically, the ratio of the liquid brightener to liquid plastic is approximately 0.1 %. The optical brightener is mixed thoroughly within the liquid plastic to ensure that the substance is uniformly dispersed within the respective medium. The optical brightener can be chosen from the group consisting of fluorescent brighteners 61, 184, and 393.

The situations should be typically avoided in which the substance with photoluminescent qualities is not distributed uniformly within a dispersing medium, but in a random fashion. In such situations certain parts of the dispersing medium or plastic are rich in the photoluminescent substance, while the other parts contain none of it. In this instance the visibility of the implement utilizing such material under UV light might diminish. This may decrease the ability of a user to see the implement or the respective portions thereof in darkness and to manipulate efficiently the DNA/RNA fragments or other biological samples. It should be noted however, that in the alternative embodiments of the invention, only predetermined or restricted areas of the implement, such as the blade and/or spatula are produced containing the required quantities of photoluminescent material. Such arrangement is typical when the respective part of the implement are formed separately and joined together by the conventional joining techniques.

In another embodiment the body 12 can be formed utilizing umbelliferone as a photoluminescent substance used to enhance visualizing the implement. This substance is known for its high melting point and widespread availability. Umbelliferone absorbs strongly at 300, 305 and 325 nm and emits visible blue light when subjected to either ultraviolet or visible light. The powerful absorption at three different wavelengths, coupled with the fact that the energy is dissipated safely as visible light, make umbelliferone useful for the method of the invention.

In a further embodiment the body 12 can be provided with an adhesive coating formulated with photo initiators. When exposed to the UV light, polymerization occurs, and the adhesives harden and cure to the tool. The coating may be placed on parts of the implement, such as the blade 26 or spatula 22.

Although the photoluminescent substances in the form of the above discussed optical brighteners and umbelliferone have been discussed hereinabove, it should be noted that use of other photoluminescent substances also contemplated for use in the implement of the invention.

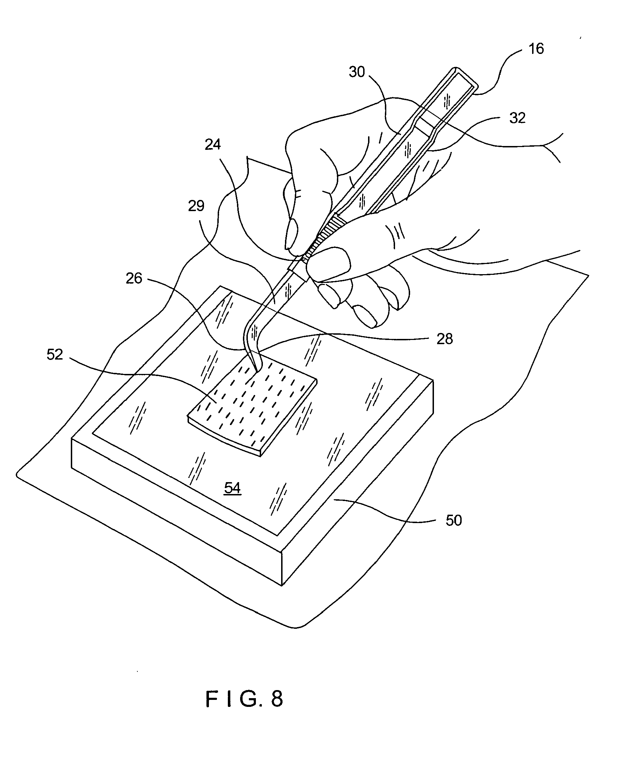

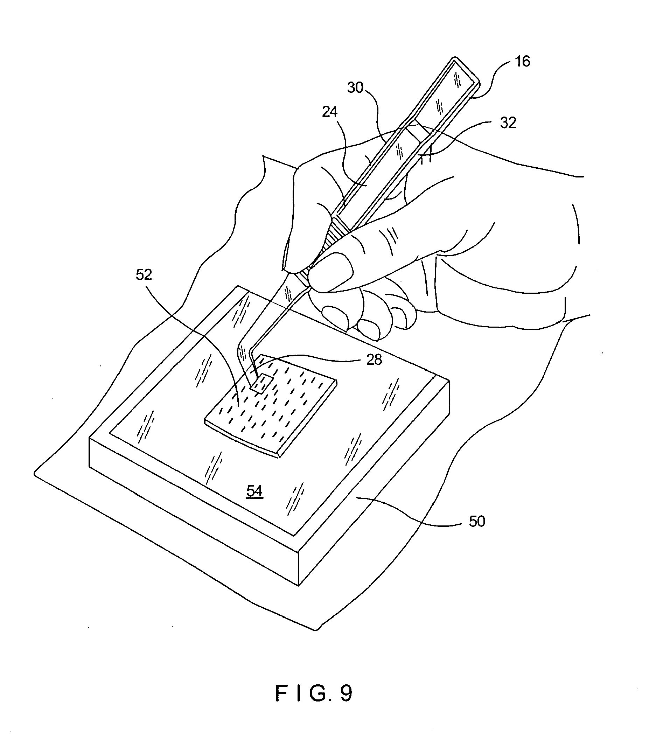

Application of the method of the invention will be discussed hereinbelow referring to the FIGs 8 and 9. In the method of the invention the implement 10 is adapted to remove and manipulate the biological samples in general and specifically to manipulate nucleotide-based fragments, such as RNA, DNA, and proteins, from agarose, acrylomide, and other colloidal gel structures. The ability of the operator to see certain parts of the implement, such as the blade 26 and/or spatula 22 during manipulation is essential to the accuracy of the procedure. This is especially so in view of the fact that nucleotide fragments on the gel structures are small and are barely visible under normal lighting conditions. The accuracy required in the method is substantially enhanced by utilizing the implement 10 of the invention formed containing photoluminescent material aimed at aiding in maneuvering the instrument by the operator under UV light without the visible light.

The implement is provided with a low rise body 12, so that it may be held substantially horizontally when used for cutting. Thus, the view of the user is unobstructed. When working inside or near the transilluminator or other apparatuses, the user reaches horizontally, so as to not have to worry about tricky manipulation. Further, the user need not fear scratching the expensive glass plate or filter below the agar gel because the implement 10 is made of resilient plastic.

The method of handling biological samples is conducted in the following manner. After the step of electrophoresis, the fragments are strewn across the surface of the gel structure and difficult to see in normal light. The gel with the fragments is viewed in ultraviolet light, usually by means of a transilluminator which provides bright and even light. The bioluminescent properties of the UV-stained nucleotides cause them to be highly visible to the naked eye or to a camera used to take a picture of the layout of the fragments on the gel.

In many instances it is desired to remove some or all of nucleotide-based fragments from the colloidal gel structure for further manipulation. The fragments are teased, pried, cut from, or otherwise acted upon to separate them from the gel. As illustrated in FIG 8 the implement 10 is positioned over a gel surface 52 which is disposed directly or indirectly on the screen 54 of the transilluminator 50. In this manner at least the gripping 24 and the transition 29 portions of the implement are disposed at an acute angle or in a parallel manner to the surface of the gel 52 with the blade 26 extending substantially vertically. In this position, as illustrated in FIG 8, the transition and gripping portions are typically disposed in a hand of a user in such a manner that the bottom edge 32 is held against the middle finger, the top edge 30 is held against an index finger with a thumb engaging a gripping portion 24.

It is further illustrated in FIG 8, that the distal end 16 of the implement is positioned in the palm area at the merger between the index finger and the thumb. According to the method of the invention, the implement is positioned in the hand of the user so that the blade 26 faces the substance containing biological samples. Although the above-described method of handling the implement is illustrated, it is to be understood that any other ways of handling the instrument are within the scope of the invention.

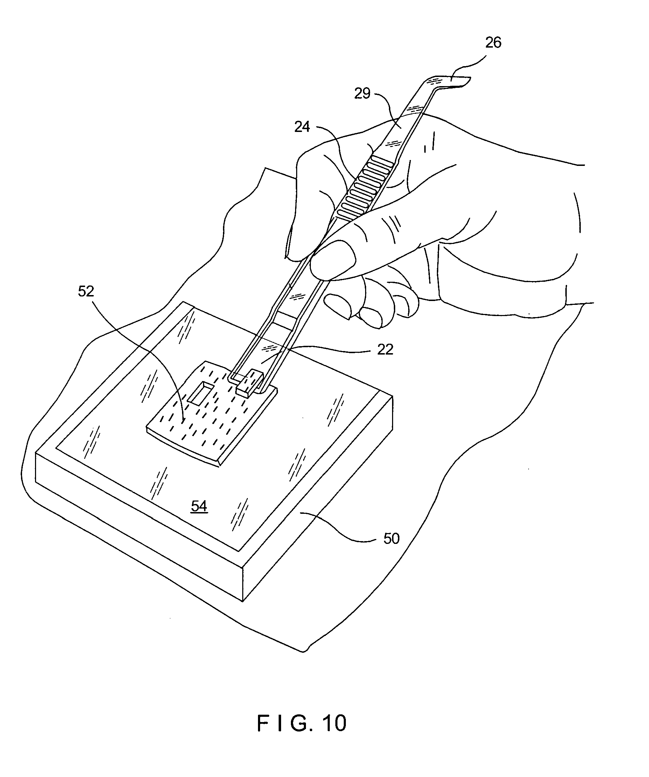

Position of the implement in general and the blade in particular with respect to the biological samples is verified in view of the ability of the user to see at least the blade 26 in darkness under the presence of UV light. Then, the substance containing the biological samples is cut utilizing the cutting region 28 and the fragments are pulled away. In one application of the step of cutting, pressure can be applied by the index finger of the hand of the user on the top edge 30 of the implement. As illustrated in FIG. 8-10, with the aid of the blade 26, one cuts the agar around the DNA band. Then the tip 27 of the blade is inserted into the agarose piece, from its side and the piece is lifted. In some instances, as shown in Fig. 10, the cut piece of agarose might accidentally fall onto the surface of the gel. Therefor, an operator can use the spatula 22 to collect the piece of gel and transfer it to a test tube. In this manner, spatula eliminates contacts between the cut fragments of gel and the surface of the gel to avoid any further contamination by the other DNA bands.

Since the spatula 22 emits visible light when subjected to UV radiation: Therefore, in the step of handling the gel with nucleotide-based fragments, the spatula and the gel (containing particles capable of emitting visible light when subjected to ultraviolet light) are visible to an operator. This feature substantially enhances precision and accuracy of the manipulation step.

The present invention solves many prior art problems by providing a single-use implement made from plastic thereby decreasing or substantially eliminating a risk of contamination. The implement 10 can be certified nuclease free signifying that its contents are free of DNA, RNA, and other nucleotides. Such a designation certifies that they device of the invention will not contaminate or mix new nucleic acids with those on the agarose gel.

## Claims

1. A method of handling biological samples by means of an implement consisting of a body formed with at least a blade at one end thereof, at least said blade having photoluminescent qualities and is capable of emitting visible light when subjected to ultraviolet light, said method is conducted in darkness under ultraviolet light, the method comprising the steps of:
positioning said implement in the hand of a user so that at least said blade faces a substance containing said biological samples;
viewing at least said blade emitting visible light;
cutting said biological samples by said blade emitting visible light; and
manipulating said biological samples by said implement.

2. The method according to claim 1, wherein said implement further comprises said body formed with a gripping portion, said blade is spaced from said gripping portion by a transition portion and positioned at an angle to the longitudinal axis to the gripping portion, a cutting region of said blade facing the gripping portion;
wherein in said step of positioning the implement is disposed within the hand of an operator in such a manner that said gripping and transition portions are disposed at an acute angle to a surface of the substance containing said biological sample.

3. The method according to claim 1, wherein said biological samples are nucleotide-based fragments, and said substance containing said biological samples is a gel.

4. The method according to claim 3, wherein said nucleotide-based fragments are selected from a group consisting of DNA, RNA, and proteins.

5. The method according to claim 1, wherein said biological samples contain particles emitting visible light when subjected to ultraviolet light, in said steps of cutting and manipulating said biological samples and said blade are visible to an operator.

6. The method according to claim 5, wherein a recess portion is formed within the body of the implement at an end thereof opposite to said blade, wherein in said step of manipulating said biological samples are manipulated by said recess portion.

7. The method according to claim 6, wherein said recess is a spatula, said spatula having photoluminescent qualities and capable of emitting visible light when subjected to ultraviolet light, so that in step of manipulating said spatula and said biological samples are visible to an operator.

8. The method according to claim 7, wherein at least said blade and spatula contain fluorescent particles emitting visible light when subjected to ultraviolet light.

9. The method according to claim 8, wherein said fluorescent particles are optical brighteners chosen from the group consisting of fluorescent brighteners 61, 184, and 393.

10. The method according to claim 4, wherein said implement is made of plastic material with particles having florescent qualities being dispersed within medium of said plastic material used in said blade and said spatula.

11. The method according to claim 10, wherein said plastic material is selected from the group of polymers including polystyrene.

12. The method according to claim 1, wherein said implement is DNA and RNA free.

13. The method according to claim 1, wherein said body of the implement further comprises a top longitudinal edge and bottom longitudinal edge, said edges extending longitudinally toward said blade forming a part of said gripping and transition portions,
wherein in said step of positioning, said transition and gripping portions are disposed in a hand of a user in such a manner that said bottom edge is held against a middle finger, said top edge is held against an index finger with a thumb engaging a gripping portion with a distal end of said implement with a portion of the hand defined as a merger between the index finger and the thumb.

14. The method according to claim 13, wherein in said step of cutting a pressure is being applied by the index finger on said top edge so as to manipulate said blade.

15. A non-metallic laboratory implement, comprising:
a body having at least a gripping portion and a blade spaced from each other, said blade positioned at an angle to a longitudinal axis to the gripping portion, a cutting region of the blade facing the gripping portion; and
at least one photoluminescent substance is being dispersed within a material medium of at least said blade, so as to make said blade visible under ultraviolet light.

16. The implement according to claim 15, said body further comprises a transition portion and a spatula, said transition portion is formed between said blade and said gripping portion, the cutting region of the blade is formed as a curvature extending from the transition portion to a tip area of a proximal end of a body, said spatula if formed at a distal end of the body, wherein at least one photoluminescent substance is dispersed within the material medium of said spatula, so as to make said spatula visible under ultraviolet light.

17. The non-metallic laboratory implement according to claim 16, wherein said at least one photoluminescent substance is uniformly dispersed within the material medium of the entire body, so as to make the entire body of said implement visible under ultraviolet light.

18. The non-metallic laboratory implement according to claim 17, wherein said dispersing medium is selected from the group of polymers including polystyrene.

19. The non-metallic laboratory implement according to claim 16, wherein said photoluminescent substance is an optical brightener chosen from the group consisting of fluorescent brighteners 61, 184, and 393.
